(19) Europäisches Patentamt
European Patent Office
Office européen des brevets

(11) Publication number: **0 080 478**
**B1**

# (12) EUROPEAN PATENT SPECIFICATION

(45) Date of publication of patent specification: **04.09.85**

(21) Application number: **82901631.0**

(22) Date of filing: **28.05.82**

(86) International application number: **PCT/NO82/00031**

(87) International publication number: **WO 82/04252 09.12.82 Gazette 82/29**

(51) Int. Cl.⁴: **C 07 D 257/02,** C 07 D 259/00, C 07 D 273/00, B 01 J 45/00

## (54) N-HYDROXYETHYLATED MACROCYCLIC POLYAMINES, THE PREPARATION OF SUCH AMINES AND THEIR USE.

(30) Priority: **01.06.81 NO 811840**

(43) Date of publication of application: **08.06.83 Bulletin 83/23**

(45) Publication of the grant of the patent: **04.09.85 Bulletin 85/36**

(84) Designated Contracting States: **AT BE CH DE FR GB LI LU NL SE**

(56) References cited:
**DE-A-2 803 528**
**FR-A-2 246 555**
**GB-A-1 529 150**
**US-A-4 093 615**

**Patent Abstracts of Japan Kokai No 54-90189, published 1979-07-17, Tokyo Shibaura Denki K.K.**

(73) Proprietor: **BORREGAARD INDUSTRIES LIMITED**
**N-1701 Sarpsborg (NO)**

(72) Inventor: **DALE, Johannes**
**Stasjonsveien 63**
**N-1310 Blommenholm (NO)**
Inventor: **BUOEN, Solfrid**
**Olav Magnussons vei 5**
**N-7000 Trondheim (NO)**
Inventor: **KRANE, Jostein**
**Elgesetergt. 35**
**N-7000 Trondheim (NO)**

(74) Representative: **Perry, Robert Edward et al**
**GILL JENNINGS & EVERY 53-64 Chancery Lane**
**London WC2A 1HN (GB)**

(56) References cited:
**Tetrahedron Letters 36 (1980), pp 461-510, (Great Britain); J S Bradshaw and P E Stott: "Preparation of derivatives and analogs of the macrocyclic oligomers of ethylene oxide (crown compou ds)", note pp 483 and 485.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 080 478 B1

## Description

This invention relates to novel N-hydroxyethylated macrocyclic polyamines, their use as complexing agent and the preparation of such compounds.

Macrocyclic polyethers with all or the majority of the ether oxygen atoms in 1,4-relationship are known to be good complexers for alkali and alkaline earth cations and are often called "crown ethers". The corresponding macrocyclic polamines, which are called "crown amines" or "aza-crowns", form less stable complexes with alkali and alkaline earth metal cations. The chemical affinity of amino nitrogen towards transition metal cations on the other hand is stronger. Mixed systems, polyaminoethers, are also known and show intermediate behaviour as cation complexers [Tetrahedron, *36,* 461 (1980)].

The secondary amino groups of the simplest crown amines have a functionality allowing the attachment of a side chain carrying an extra donor atom. Such "polypod" molecules have now been found to be optimally suited for cation complexing if the donor atom of the side chain is separated from the ring by a $—CH_2CH_2$-group, for the same reason that a 1,4-relationship between donor atoms in the ring is favourable for efficient cation complexing. The 2-hydroxyethyl group is of particular interest since it is electrically neutral and has a hydroxyl group that can in principle bond through lone-pair elecrons to cations, as well as through hydrogen bond formation to another anion, and thus form ion-pair complexes rather than cation complexes. The 2-hydroxyethyl groups would also restore some of the affinity to alkali and alkaline earth metal cations that is too weak in the parent polyamines.

US—A—4093615 describes tetrakis(2-hydroxyethyl)-1,4,7,10-tetraazacyclododecane and its preparation, but makes no mention of complexing properties.

It is well known that secondary amines of open-chain structure, or cyclic structure of normal ring size, can be readily hydroxyethylated with ethylene oxide under a variety of conditions, and reported examples include molecules with more than one amino function. There are also examples of macrocyclic aminoethers where it has been possible to hydroxyethylate secondary amino groups successfully under similar conditions. For example, not only does J. Org. Chem. *40* (1975) 1205 describe the hydroxyethylation of a monoaza-crown ether, but J.C.S. Chem. Comm (1976) 640, Acta Chem. Scand. *B33* (1979) 469 and 698 report the hydroxyethylation of, respectively, 4,7-dioxo-1,10-diaza-12,14-benzocyclopentadecane, 1,4,10,13-tetraoxa-7,16-diazacyclooctadecane and 1,7-dioxa-4,10-diazacyclododecane. In all these diaza-crown ethers, the two NH-functions are separated from each other in the molecule by a chain of at least 5 atoms.

We have found that closely-related 12-member ring compounds having NH-functions spaced apart by a chain of only 2 atoms do not react under the same conditions, nor is such a process disclosed in US—A—4093615 (see above). Both 1-oxa-4,7,10-triazacyclododecane and 1,4,7,10-tetraazacyclododecane were recovered unchanged after treatment with ethylene oxide in water or methanol under a variety of conditions which have been prescribed for other amines.

Novel N-hydroxyethylated cyclic polyamines according to the present invention have the formula

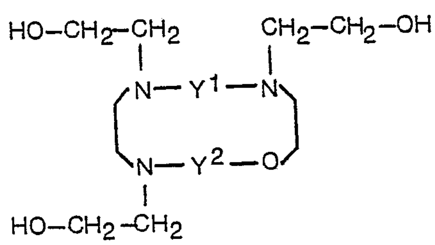

in which $Y^1$ and $Y^2$ are the same or different and are each $—CH_2—CH_2—$ or $—CH_2—CH_2—N(CH_2CH_2OH)—CH_2—CH_2—$.

These new compounds have generally substantially better complexing properties than known macrocyclic polyamines, particularly towards alkali and alkaline earth metal cations. These superior complexing properties may be used, for example, in extraction or purification.

A process according to the present invention, for preparing a N-(2-hydroxyethyl) derivative of a cyclic polyamine containing from 3 to 6 nitrogen atoms of which pairs are separated by 2 ring atoms in a heterocyclic ring, comprises neutralising an aqueous solution of the non-hydroxyethylated polyamine, in the form of an acid addition salt, with an inorganic base, extracting the polyamine thus freed from the aqueous solution with an organic solvent, and reacting the extract with ethylene oxide.

In the process of the invention, it is preferred that the freed polyamine is recovered from the organic solvent after extraction, and reacted with ethylene oxide in water. The reaction product is preferably recovered from the aqueous reaction mixture by extraction with an organic solvent. Chloroform is preferred for use as solvent for the extraction of the free polyamine or of the reaction product.

In arriving at the novel process, it was noted that polyamines are often isolated and stored as hydrochlorides or other salts, so that the amine has to be liberated for the reaction by addition of alkali hydroxide. The reason for the previously unsuccessful hydroxyethylation efforts could be that the correct pH for the reaction had not been found. If easy ring opening requires protonation of ethylene oxide, a need for the presence of free amine groups could result in a narrow pH window for the reaction.

It was then found that, for simple mono- and diamines, any lowering of the pH value by mineral acid decreased the reaction rate. Thus, using morpholine as a model in water at 0°C, 85% hydroxyethylation was obtained in 0.5 hours, whereas only 47% had reacted if morpholine was first half-neutralised with HCl. In methanol (no HCl), only 14% was hydroxyethylated under the same conditions. This shows that protonation is not necessary to activate ethylene oxide for ring opening. A possible explanation of the failure of earlier attempts to hydroxyethylate aza-crown ethers could therefore be complexation between the cation introduced with the base which was used to liberate the amine from its salt and the amine. It may be noted that the addition of NaCl to morpholine had no effect, but strong cation complexing is of course not expected here.

To examine this possible explanation further, we then dissolved the polyamine hydrochloride in concentrated KOH solution and extracted the liberated amine in salt-free form, with $CHCl_3$. Reaction with ethylene oxide then took place remarkably readily. Water turned out to be by far the best solvent for the reaction which in some cases was finished within less than an hour at 0°C. This low temperature is an advantage because of the volatility of ethylene oxide, but too high concentrations of ethylene oxide in water give crystalline clathrates [J. Chem. Phys., *42,* 2725 (1965)] which make stirring difficult. It proved essential to use only a reasonable excess (100—200%) of the stoichiometric quantity of ethylene oxide and to follow the progress of the reaction by $^{13}C$ NMR spectroscopy. When the reaction is complete, the excess ethylene oxide is evaporated in vacuo. Too long reaction time, excessive quantity of ethylene oxide, and too high temperature may lead to by-products arising from side-chain alongation. Sometimes ring opening of ethylene oxide to give ethylene glycol and polymerization to polyethylene glycols is also observed.

The hydroxylated polyamine may be isolated and purified by crystallization as such, or through formation of crystalline salt complexes. Further, extraction with $CHCl_3$ is also effective. Presumably the more fully hydroxyethylated the products are, the more lipophilic they become due to internal hydrogen bonding of each OH group to its nearest tertiary amine function. At least, the completely hydroxyethylated products go preferentially into the $CHCl_3$ phase, whereas incompletely hydroxyethylated and chain-elongated products tend to remain preferentially in the aqueous phase. Instead of chloroform as extracting agent for the extraction of the free amine base from the salt and/or for the extraction of the hydroxyethylated product from the reaction mixture, it is also possible to use other organic solvents, such as dichloromethane.

The present process is particularly suitable for the hydroxyethylation of macrocyclic amines having three or more nitrogen atoms in the ring, particularly such in which there is only one dimethylene group between the amino functions, i.e. a 1,4-relationship. The process is characterized by the features of the process claims.

In the following examples reference is made to formulae at the end of the description. "Rotavapor" may be a registered Trade Mark.

Example 1

Tetrakis-(2-hydroxyethyl)-1,4,7,10-tetraazacyclododecane

The tetratosyl derivative *Id* was synthesized according to Richman and Atkins (J. Am. Chem. Soc., *96,* 2268 (1974)), detosylated in sulfuric acid and isolated as the tetrahydrochloride *Ic* (J. Am. Chem. Soc., *96,* 2268 (1974)). The free amine *Ib* was obtained by dissolving the tetrahydrochloride in 1M aqueous KOH and extraction with chloroform. The chloroform was evaporated and the free amine *Ib* recovered.

The amine *Ib* (0.97 g) was dissolved in water (10 ml) at 0°C. A solution of ethylene oxide (2.35 g) in water (2 ml) cooled to 0°C was added, and the cooled mixture was stirred for 2 hours. The reaction was stopped by evaporation of the excess of ethylene oxide in a Rotavapor. After further concentration at room temperature, the hydroxyethylated product *Ia* crystallized (m.p. 90—92°C, 1.02 g = 52%).

$^{13}C$ NMR in $H_2O$: δ (ref. $CH_3CN$) 48.9 (8 ring $NCH_2$), 55.0 (4 side chain $NCH_2$), 57.6 (4 $OCH_2$).

MS, CI (isobutane): 349 (M+1)

$C_{16}H_{36}N_4O_4{\cdot}H_2O$ requires: C 52.4, H 10.5

Found: C 52.5, H 10.3.

The actual yeild of *Ia* is much higher as judged from the $^{13}C$ NMR spectrum of the crude reaction product.

Titration of the pure compound *Ia* in methanol by portion addition of dry salt, monitored by $^{13}C$ chemical shift, shows that strong 1:1 complexes are formed with $LiClO_4$ and NaSCN and a much weaker with KSCN. With $Ca(NO_3)_2$ and $Sr(NO_3)_2$ very strong 1:1 complexes are formed which show slow exchange with excess ligand in $^{13}C$ NMR.

Example 2

Tris-(2-hydroxyethyl)-1-oxa-4,7,10-triazacyclododecane

The triamine *IIb* was prepared and isolated in the same way as described for *Ib.* Hydroxyethylation was

carried out as before on a solution of the triamine *IIb* (1.23 g) and ethylene oxide (2.90 g) in water (11 ml) for 2 hours. The MS and $^{13}C$ NMR spectra showed that the expected compound *IIa* was the main product. A crystalline complex with NaSCN was prepared from ethanol solution (m.p. 154—170°C, 1.25 g = 45%).
$^{13}C$ NMR in $D_2O$: δ (ref. $CH_3CN$) 48.5, 50.6, 54.4, 54.8, 57.3, 65.7.
MS, CI (isobutane): 306 (M + 1 of ligand).

$C_{15}H_{31}N_4O_4SNa$ requires: C 46.6, H 8.1
Found: C 46.7, H. 8.1.

The actual yield of *IIa* is much higher as judged from the $^{13}C$ NMR spectrum of the crude reaction product.

Example 3

Pentakis-(2-hydroxyethyl)-1,4,7,10,13-pentaazacyclopentadecane

The pentaamine *IIIb* was prepared and isolated as described for *Ib.* The free amine *IIIb* (0.10 g) was dissolved in water (2.6 ml) at 0°C, and ethylene oxide (0.23 ml), precooled to −78°C, added. After stirring for 30 hours at 0°C, the excees of ethylene oxide was evaporated in a Rotovapor. The $^{13}C$ NMR spectrum showed that compound *IIIa* was the main product, with the three expected strong lines in $D_2O$ at δ (ref. $CH_3CN$) 49.5, 55.1 and 58.0. Distribution between chloroform and water gave a clear enrichment of *IIIa* in the chloroform layer and of accompanying by-products in the water layer. An isolated yield of *IIIa* from the chloroform layer was 33%, but the actual yield is much higher.

Example 4

Hexakis-(2-hydroxyethyl)-1,4,7,10,13,16-hexaazacyclooctadecane

The hexamine *IVb* liberated with $BaCO_3$ from the commercially available tris-dihydrosulfate *IVc* and extracted with chloroform. The free amine (0.1C g) was hydroxyethylated for 50 hours as described for *IIIb,* using ethylene oxide (0.23 ml) in water (5.6 ml). The $^{13}C$ NMR spectrum showed that compound *IVa* was the main product, with the three expected strong lines in $D_2$ at δ (ref. $CH_3CN$) 49.9, 54.7 and 57.9. Distribution between chloroform and water gave a clear enrichment of *IVa* in the chloroform layer and of accompanying byproducts in the water layer. An isolated yield of *IVa* from the chloroform layer was 16%, but the actual yield is much higher.

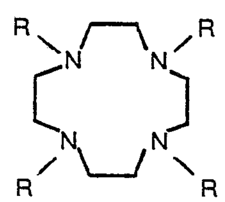

I

a 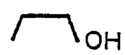

b R = H

c R = H·HCl

d R = Tos

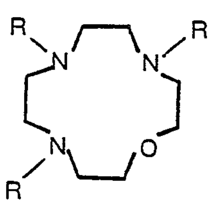

II

a 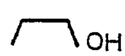

b R = H

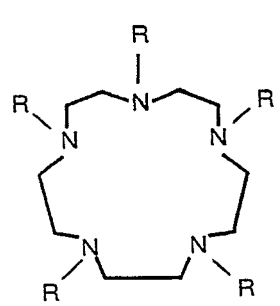

III

a 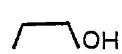

b R = H

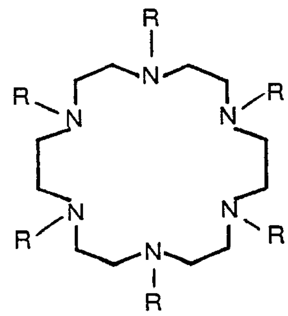

IV

a 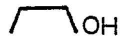

b R = H

c $R = H \cdot \frac{1}{2}H_2SO_4$

## Claims

1. A N-hydroxyethylated cyclic polyamine of the formula

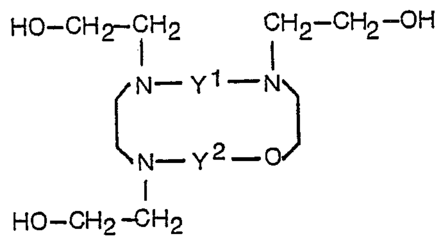

in which $Y^1$ and $Y^2$ are the same or different and are each

$$—CH_2—CH_2— \text{ or } —CH_2—CH_2—N(CH_2CH_2OH)—CH_2—CH_2—.$$

2. 4,7,10-Tris(2-hydroxyethyl)-1-oxa-4,7,10-triazacyclododecane.

3. The use of a polyamine according to claim 1 or claim 2, as a complexing agent for cations.

4. The use according to claim 3, in which the cations are alkali or alkaline earth metal ions.

5. A process for preparing a N-(2-hydroxyethyl) derivative of a cyclic polyamine containing from 3 to 6 nitrogen atoms of which pairs are separated by 2 ring atoms in a heterocyclic ring, which comprises reacting the non-hydroxyethylated cyclic amine with ethylene oxide, characterised in that an aqueous solution of the non-hydroxyethylated polyamine, in the form of an acid addition salt, is neutralised with an inorganic base, and the polyamine thus freed is extracted from the aqueous solution with an organic solvent, before reaction with the ethylene oxide.

6. A process according to claim 5, in which the freed polyamine is recovered from the organic solvent after extraction and reacted with ethylene oxide in water.

7. A process according to claim 6, in which the reaction product is recovered from the aqueous reaction mixture by extraction with an organic solvent.

8. A process according to any of claims 5 to 7, in which chloroform is used as solvent for the extraction of the free polyamine or of the reaction product.

9. A process according to any of claims 5 to 7, in which the product is as claimed in claim 1 or claim 2.

## Patentansprüche

1. N-hydroxyethyliertes cyclisches Polyamin der Formel

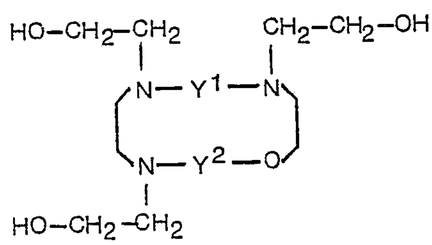

worin $Y^1$ und $Y^2$ gleich oder verschieden sind und jeweils für

$$-CH_2-CH_2- \text{ oder } -CH_2-CH_2-N(CH_2CH_2OH)-CH_2-CH_2-$$

stehen.

2. 4,7,10-Tris(2-hydroxyethyl)-1-oxa-4,7,10-triazacyclododecan.

3. Verwendung eines Polyamins nach Anspruch 1 oder 2 als Komplexierungsmittel für Kationen.

4. Verwendung nach Anspruch 3, worin die Kationen Alkali- oder Erdalkalimetallionen sind.

5. Verfahren zur Herstellung eines N-(2-Hydroxyethyl)-Derivats eines cyclischen Polyamins mit 3 bis 6 Stickstoffatomen, von welchem Paare durch 2 Ringatome in einem heterocyclischen Ring getrennt sind, welches Verfahren ein Umsetzen des nichthydroxyethylierten cyclischen Amins mit Ethylenoxid umfaßt, dadurch gekennzeichnet, daß eine wässerige Lösung des nichthydroxyethylierten Polyamins, in der Form eines Säureadditionssalzes, mit einer anorganischen Base neutralisiert und das solcherart freiegesetzte Polyamin aus der wässerigen Lösung mit einem organischen Lössungsmittel extrahiert wird, bevor die Umsetzung mit dem Ethylenoxid erfolgt.

6. Verfahren nach Anspruch 5, worin das freigesetzte Polyamin aus dem organischen Lösungsmittel nach der Extraktion gewonnen und mit Ethylenoxid in Wasser umgesetzt wird.

7. Verfahren nach Anspruch 6, worin das Reaktionsprodukt aus dem wässerigen Reaktionsgemisch durch Extraktion mit einem organischen Lösungsmittel gewonnen wird.

8. Verfahren nach einem der Ansprüche 5 bis 7, worin Chloroform als Lösungsmittel für die Extraktion des freien Polyamins oder des Reaktionsproduktes verwendet wird.

9. Verfahren nach einem der Ansprüche 5 bis 7, worin das Produkt eine Verbindung ist, wie in Anspruch 1 oder Anspruch 2 beansprucht.

**Revendications**

1. Polyamine cyclique N-hydroxyéthylée, caractérisée en ce qu'elle répond à la formule

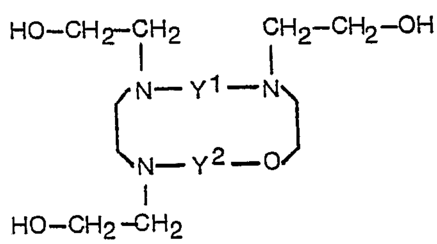

dans laquelle $Y^1$ et $Y^2$ sont identiques ou différents et représentent chacun

$$-CH_2-CH_2- \text{ ou } -CH_2-CH_2-N(CH_2CH_2OH)-CH_2-CH_2-.$$

2. Le 4,7,10-tris(2-hydroxyéthyl)-1-oxa-4,7,10-triazacyclododécane.

3. Utilisation d'une polyamine selon la revendication 1 ou 2 comme agent complexant pour des cations.

4. Utilisation selon la revendication 3, dans laquelle les cations sont des ions de métaux alcalins ou alcalinoterreux.

5. Procédé pour la fabrication d'un dérivé N-(2-hydroxyéthylé) d'une polyamine cyclique contenant de 3 à 6 atomes d'azote dont les paires sont séparées par 2 atomes du cycle dans un noyau hétérocyclique, qui comprend la réaction de l'amine cyclique non hydroxyéthylée avec l'oxyde d'éthylène, caractérisé en ce que l'on neutralise par une base inorganique une solution aqueuse de la polyamine non hydroxyéthylée, sous la forme d'un sel d'addition d'acide, et on extrait de la solution aqueuse par un solvant organique la polyamine ainsi libérée, avant la réaction avec l'oxyde d'éthylène.

6. Procédé selon la revendication 5, caractérisé en ce que la polyamine libérée est récupérée du solvant organique après extraction et mise à réagir avec l'oxyde d'éthylène dans l'eau.

7. Procédé selon la revendication 6, caractérisé en ce que le produit de réaction est récupéré du mélange de réaction aqueux par extraction par un solvant organique.

8. Procédé selon l'une quelconque des revendications 5 à 7, caractérisé en ce que l'on utilise le chloroforme comme solvant pour l'extraction de la polyamine libre ou du produit de réaction.

9. Procédé selon l'une quelconque des revendications 5 à 7, caractérisé en ce que le produit est un produit selon la revendication 1 ou 2.